# EUROPÄISCHE PATENTSCHRIFT

(11) **EP 3 682 453 B1**
(45) Veröffentlichungstag und Bekanntmachung des Hinweises auf die Patenterteilung: **24.01.2024**
(21) Anmeldenummer: 18765883.6
(22) Anmeldetag: 07.09.2018
(51) Int. Cl.: G16H 50/50, A61C 7/00

(54) **VERFAHREN ZUR ERMITTLUNG UND VISUALISIERUNG VON ZAHNBEWEGUNGEN UND GEPLANTEN ZAHNUMSTELLUNGEN**
METHOD FOR DETERMINING AND VISUALIZING TOOTH MOVEMENTS AND PLANNED INSTANCES OF TOOTH REPOSITIONING
PROCÉDÉ DE DÉTERMINATION ET DE VISUALISATION DE MOUVEMENTS DE DENTS ET DE CHANGEMENTS DE POSITION DE DENTS PREVUS

(30) Priorität: 15.09.2017 DE 102017121451
(43) Veröffentlichungstag der Anmeldung: 22.07.2020
(73) Patentinhaber: SICAT GmbH & Co. KG, 53175 Bonn (DE)
(72) Erfinder: HANßEN, Nils, 53125 Bonn (DE)
(74) Vertreter: Braun-Dullaeus Pannen Emmerling Patent- & Rechtsanwaltspartnerschaft mbB
(86) Internationale Anmeldenummer: PCT/EP2018/074142
(87) Internationale Veröffentlichungsnummer: WO 2019/052915

(56) Entgegenhaltungen:
- US-A1- 2012 015 316

## Beschreibung

Die Erfindung betrifft ein Verfahren zur Darstellung einer Veränderung einer Zahnstellung in einem Kiefer, wobei im Rahmen der Verfahrensweise ein erster Volumendatensatz umfassend eine Ausgangsstellung mehrerer Zähne im Ober- oder Unterkiefer angefertigt wird.

Bekanntermaßen werden heutzutage kieferorthopädische Behandlungen, die mit der Umstellung von Zähnen einher gehen, anhand der sichtbaren Zahnanteile geplant und durchgeführt. Dazu werden im Rahmen der Planung und der späteren Kontrolle konventionelle Gipsmodelle, insbesondere das sogenannte kieferorthopädische Set Up, herangezogen, wobei im Zuge der Planung ein Gipsabdruck der Ist-Situation erstellt wird, aus dem dann einzelne Zähne oder Zahngruppen herausgesägt und mit Wachs in der neuen "idealen" Position zusammengefügt werden. Mit einem solchen kieferorthopädischen Set Up kann eine Behandlung gewissermaßen simuliert werden.

Neben dieser konventionellen Art der Planung anhand von Gipsmodellen werden mitunter auch digitale Oberflächendaten mit optischen Scannern aufgenommen, um diese Daten in die Planung einer mit der Therapie zu erreichenden Zielstellung einbeziehen zu können. Solches ist beispielsweise aus dem US 2012/0015316 A1 bekannt. Auch die DE 10 2007 001 684 A1 sind Verfahrensweisen zur Bildregistrierung bekannt.

Keines der bislang im Bereich der Kieferorthopädie bekannten Planungsverfahren berücksichtigt jedoch die Auswirkungen der geplanten Umstellung auf die Zahnwurzeln und auf die Kiefergelenke. Damit fehlt den bekannten Verfahren jeglicher Bezug zu der im Kiefer verborgenen, knöchernen Anatomie. Da die anatomischen Gegebenheiten jedoch die Möglichkeiten einer geplanten kieferorthopädischen Umstellungsmaßnahme stark beeinflussen können, ist deren Berücksichtigung für den behandelnden Arzt von großem Interesse.

Aufgabe der Erfindung ist es daher, ein einfach und kostengünstig umzusetzendes Verfahren vorzuschlagen, mit dem sich die individuellen anatomischen Gegebenheiten eines Patienten bei der Planung einer kieferorthopädischen Umstellungsmaßnahme auf komfortable Weise berücksichtigen lassen. Zudem ist es die Aufgabe, ein entsprechendes System zur Durchführung des Verfahrens zur Verfügung zu stellen.

Diese Aufgaben werden durch das Verfahren nach Anspruch 1 und das System nach Anspruch 10 gelöst. Bevorzugte Ausgestaltungen ergeben sich aus den jeweiligen Unteransprüchen.

Anspruchsgemäß liegt der Kern der Erfindung zusammengefasst darin, dass nach einer realen Veränderung der Zahnstellung am Patienten oder einer (quasi virtuellen oder modellierten) im Zuge einer Planung vorgenommenen Veränderung der Zahnstellung an einem Modell ein optischer Oberflächendatensatz erstellt wird und dass ein am Patient erstellter Volumendatensatz entsprechend dieser Oberflächendaten, welche die Umstellung charakterisieren, rechnerisch zu einem modifizierten Volumendatensatz modelliert wird. Dazu wird der die veränderte Situation beschreibende Oberflächendatensatz mit den tomographischen Daten zur Deckung gebracht, mithin registriert. Bei dieser Vorgehensweise können die einzelnen Zähne in den optischen Oberflächendaten isoliert und einzeln zur Deckung gebracht werden. Durch die unterschiedlichen Transformationen der Einzelzahnanteile auf die tomographischen Daten werden die relativen Transformationen der Zähne offenbar. Die Transformation jedes einzelnen Zahnes kann dann auf den im Tomogramm sichtbaren Zahn mitsamt seiner Wurzel angewendet werden.

Erfindungsgemäß sind unterschiedliche Szenarien zur Erstellung des Oberflächendatensatzes möglich. So kann der Oberflächendatensatz mit einem optischen Scanverfahren erzeugt werden, wobei entweder die tatsächlich veränderte Zahnstellung im Mund des Patienten oder aber die modellierte Veränderung an einem Oberflächenmodell aufgenommen wird. Es ist aber auch möglich, den Oberflächendatensatz der veränderten Zahnstellung rechnerisch (virtuell) aus einem vorhandenen Oberflächendatensatz der ursprünglich noch nicht veränderten Zahnstellung am Rechner durch Simulation zu erzeugen.

Anhand des erfindungsgemäß erstellten modifizierten Volumendatensatzes lässt sich dann die anatomische Auswirkung der geplanten oder der schon bis zu diesem Zeitpunkt erfolgten Umstellung ersehen. Dieser durch rechnerische Simulation erstellte modifizierte Volumendatensatz macht es möglich, dass Änderungen bei der Planung oder während der Therapie vorgenommen werden, wobei in jedem Fall ein einziger Volumendatensatz, der an der Ursprungsstellung der Zähne aufgenommen wurde, für diese Vorgehensweise ausreicht.

Anspruchsgemäß drückt sich die erfindungsgemäße Vorgehensweise darin aus, dass mit einem optischen Scanverfahren ein Oberflächendatensatz entweder an einer real erfolgten Veränderung der Zahnstellung an den Zähnen eines Patienten oder an einer virtuellen respektive modellierten Veränderung der Zahnstellung an einem Oberflächenmodell angefertigt wird, wobei der Oberflächendatensatz Oberflächendaten von Zähnen und/oder von Teilen des unveränderlichen Kiefers bei veränderter Zahnstellung enthält. Der Volumendatensatz, der im Fall der am Modell simulierten Umstellung auch zu einem späteren Zeitpunkt aufgenommen werden kann, umfasst ebenfalls diese Teile des unveränderlichen Kiefers. Die Oberflächendaten des unveränderlichen Teiles des Kiefers dienen als Referenzstruktur, mit der die beiden unterschiedlichen Datensätze miteinander registriert werden. Entsprechend wird der Oberflächendatensatz vermittels dieser im Oberflächendatensatz ersichtlichen Referenzstruktur, die von der Veränderung nicht betroffen ist, mit dem Volumendatensatz, aus dem diese Referenzstruktur rechnerisch "herauspräpariert" wird, referenziert.

Wenn der Oberflächendatensatz mit dem Volumendatensatz referenziert ist, wird die Veränderung der Zahnstellung rechnerisch in den Volumendatensatz transformiert, indem aus dem Volumendatensatz ermittelte Grenzflächen der Zähne mit Oberflächendaten der entsprechenden Zähne aus dem Oberflächendatensatz in Deckung gebracht werden. Zusammen mit diesem "in Deckung bringen" der aus dem Volumendatensatz ersichtlichen Zahnoberflächen mit den aus den entsprechenden Zahnoberflächen aus dem Oberflächendatensatz werden zugleich die starr mit den Zahnoberflächen verbundenen unsichtbaren weil unter der Oberfläche verborgenen Zahnstrukturen, wie die Wurzeln und die im Zahnfleisch befindlichen Zahnhälse, im Volumendatensatz rechnerisch entsprechend mitbewegt. Auf diese Weise entsteht ein modifizierter (simulierter) Volumendatensatz, der zur Darstellung ausgegeben werden kann und der die rechnerisch ermittelte Veränderung der Zahnstellung umfasst. Dieser simulierte Volumendatensatz erlaubt dem behandelnden Arzt Einblicke in die individuellen anatomischen Gegebenheiten des Patienten, die er entweder im Rahmen der Planung einer kieferorthopädischen Umstellungsmaßnahme berücksichtigen kann oder die ihm nach einer schon erfolgten Zahnumstellung die anatomischen Gegebenheiten vor Auge führen, ohne dass die Anfertigung eines neuen Volumendatensatzes notwendig ist.

Mit der erfindungsgemäßen Vorgehensweise lassen sich somit in einem ersten Anwendungsfall die während der Therapie vollzogenen Zahnbewegungen auch im Hinblick auf die Kieferanatomie im Sinne einer Verlaufskontrolle visualisieren. Diese Verlaufskontrolle kann der behandelnde Kieferorthopäde mehrmals während der Behandlung durchführen, um eventuell Korrekturen an der Behandlung vorzunehmen. Für jede Kontrolle ist jeweils nur die Erstellung eines Oberflächenscans notwendig, der in der kieferorthopädischen Praxis durchgeführt werden kann. Der jeweilige Oberflächenscan dient dann als Grundlage für die rechnerische Erstellung eines entsprechenden simulierten Volumendatensatzes.

In einem zweiten Anwendungsfall lassen sich Zahnumstellungen der erfindungsgemäßen Vorgehensweise schon im Vorfeld unter Berücksichtigung der verborgenen Kieferanatomie planen. Dazu erstellt der behandelnde Arzt ein Modell der späteren Zahnstellung mit Hilfe eines kieferorthopädischen Set Ups und fertigt einen Oberflächenscan von dem Modell an. In diesem Anwendungsfall dient der Oberflächenscan an dem Modell als Grundlage für die rechnerische Erstellung des entsprechenden simulierten Volumendatensatzes, an dem der behandelnde Arzt erkennen kann, welche anatomischen Auswirkungen seine geplante Zahnumstellung haben würde.

Nachfolgend wird die Erfindung an mehreren Ausführungsbeispielen anhand der Figuren 1 bis 5 beschrieben. Es zeigen:
- **Figur 1**: eine schematisierte anatomische Situation im Kiefer eines Patienten,
- **Figur 2**: das schematisierte Ergebnis eines optischen Oberflächenscans der anatomischen Situation,
- **Figur 3**: das schematisierte Ergebnis einer DVT Aufnahme der anatomischen Situation,
- **Figuren 4a-c**: ein Schema der Ausführungsform mit zwei Oberflächenscans und
- **Figuren 5a, 5b**: ein Schema der Ausführungsform mit einem Oberflächenscan.

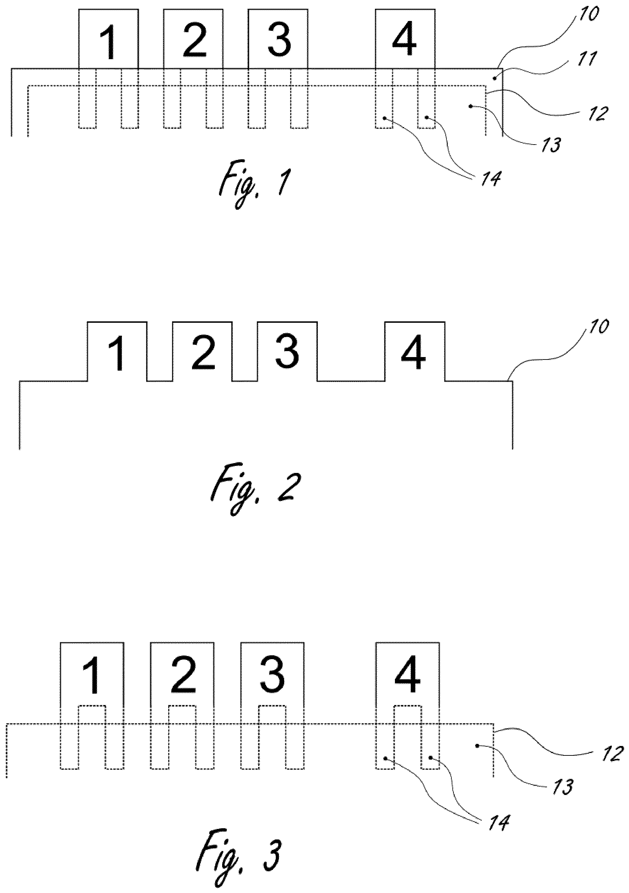

Figur 1 zeigt zunächst das Schema einer anatomischen Ausgangssituation im Kiefer eines Patienten, in dem vier Zähne 1, 2, 3, und 4 mit teilweise unterschiedlichen Abständen angeordnet sind. Dargestellt ist zum einen die Oberfläche 10 der Kieferschleimhaut 11, die eine Grenzfläche hin zur Luft bildet. Auf der anderen Seite bildet die Kieferschleimhaut 11 eine Grenzfläche 12 zum Kieferknochen 13 aus, in dem die Zahnwurzeln 14 sitzen.

In der Figur 2 ist das Ergebnis eines optischen Oberflächenscans an dem Kiefer aus Figur 1 dargestellt. Ersichtlich werden von dem Oberflächenscan lediglich die sichtbaren Oberflächen der Zähne 1 bis 4 und die Oberfläche 10 der Kieferschleimhaut erfasst.

Im Gegensatz dazu zeigt Figur 3 das Ergebnis einer DVT Aufnahme, die mittels eines Digitalen Volumen Tomographen, beispielsweise einem Cone Beam CT, aufgenommen wurde. In einem solchen dreidimensionalen Datensatz lassen sich zwar die harten Strukturen unter den Oberflächen darstellen; die Grenzen weichen Gewebes können hingegen nur schwerlich aufgelöst werden. Insofern sind in einer solchen DVT Aufnahme die harten Zähne 1 bis 4 mit ihren Wurzeln 14, der Kieferknochen 13 und die Grenzfläche 12 zwischen Kieferknochen und Kieferschleimhaut zu erkennen.

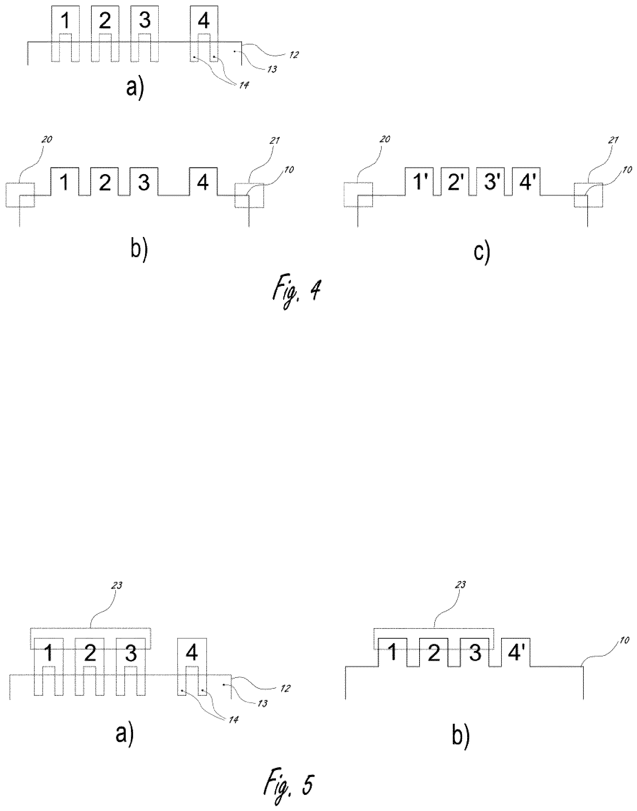

Das erste zu beschreibende Ausführungsbeispiel betrifft den ersten Anwendungsfall der anatomischen Verlaufskontrolle einer kieferorthopädischen Zahnumstellung, wobei in diesem Fall die Zahnumstellung mit einer herausnehmbaren KFO-Apparatur, beispielsweise einer herkömmlichen Zahnspange, erfolgt. Das Ausführungsbeispiel wird anhand der Figuren 4 beschrieben. Dieses Ausführungsbeispiel ist besonders relevant für Fälle, in denen lediglich Weichgewebe als Referenzstruktur dienen kann, weil sich alle Zähne bewegt haben. Da die Referenzstrukturen in den DVT-Daten nicht sichtbar sind, wird ein zweiter optischer Oberflächendatensatz benötigt.

Für dieses erste Ausführungsbeispiel werden zu Beginn der Therapie zu einem Zeitpunkt T0 eine DVT Aufnahme am Patienten und ein Satz von Oberflächendaten am Patienten oder am Modell erstellt. Aus Figur 4a ist ersichtlich, dass die Zähne 1 bis 4 eine ursprüngliche Position einnehmen. In der DVT Aufnahme sind die Wurzeln 14 und die Grenzfläche 12 des Kieferknochens zu erkennen. Figur 4b zeigt dieselbe Situation zum Zeitpunkt T0 mit einem optischen Scanner aufgenommen. Figur 4c stellt hingegen die Situation nach einer therapeutischen Verschiebung der Zähne 1 bis 4 zu einem Zeitpunkt T1 dar. Die in den Oberflächendaten erkennbaren Bereiche 20 und 21 der Kieferschleimhaut haben sich anders als die Zähne 1 bis 4 nicht verändert und können daher als feste Referenzstruktur genutzt werden.

Dieses Ausführungsbeispiel geht entsprechend mit folgenden Schritten einher, die nicht zwingend in dieser Reihenfolge abzuarbeiten sind:
1. Im Vorfeld der Behandlung - zur Zeit T0 - wird insbesondere im Rahmen einer Zahnstatusaufnahme einerseits ein DVT-Datensatz DVT_T0 und andererseits ein erster optischer Oberflächendatensatz OO_T0 eines Patienten aufgenommen.
2. Wenn die Behandlung zu einem Zeitpunkt T1 fortgeschritten ist, wird der okklusale Status des Patienten mit einer Intraoralkamera gescannt und damit der weitere Oberflächendatensatz OO_T1 aufgenommen, wobei diese Aufnahme ohne eine KFO-Apparatur im Mund des Patienten geschieht.
3. Nachfolgend wird die Registrierung zwischen den Datensätzen DVT_T0 und OO_T0 Datensatz durchgeführt. Da beide Datensätze den gleichen unveränderten Zahnstatus zum Zeitpunkt T0 abbilden, können sie anhand der Zahnkronen registriert werden.
4. Dem folgt die Registrierung zwischen den Datensätzen OO_T0 und OO_T1, bei der Bereiche entlang des Zahnbogens identifiziert werden, die sich nicht verändert haben. Anhand dieser festen Referenzstrukturen werden die OO_T0-Daten mit den OO_T1-Daten oder umgekehrt zur Deckung gebracht.
5. Im nächsten Schritt werden nun einzelne Bereiche respektive Teile im OO_T0 Datensatz betrachtet und durch eine "teilrigide" Registrierung zur Deckung mit den OO_T1-Daten gebracht. Dabei kann auf eine Segmentierung des OO_T0-Datensatzes zurückgegriffen werden. Die Segmentierung definiert, welche Teile der Oberflächendaten OO_T0 als eigenständige Objekte aufgefasst werden und diese nun einzeln ("teilrigide") mit den OO_T1 Oberflächendaten zur Deckung gebracht werden können. So kann die Segmentierung beispielsweise jeden einzelnen Zahn als eigenständiges Objekt ausweisen.
6. Durch die in Schritt 5) durchgeführte teilrigide Registrierung ist für jeden (segmentierten) Zahn respektive für jede Zahnkrone in den OO_T0-Daten bekannt, wie sich dieser Zahn im Rahmen der Therapie zwischen den Zeitpunkten T0 und T1 bewegt hat.
7. Nun werden die identifizierten Bewegungen der Zahn(kronen) auf die DVT_T0-Daten übertragen. Das ist möglich, weil durch die Registrierung zwischen DVT_T0 und OO_T0 die räumlichen Beziehungen bekannt sind. Beispielsweise sei ein bestimmter Zahn zwischen dem Zeitpunkt T0 und T1 entsprechend der Transformation Transf_T0_T1 bewegt worden. Diese Transformation wird nun auf das knöcherne Gewebe in den DVT_T0-Daten unterhalb der Zahnkrone übertragen und visualisiert. Durch Verlängerung der Zahnkrone nach unten kann das entsprechende Wurzelgewebe des Zahnes ermittelt werden. Alternativ kann das Wurzelgewebe durch eine Segmentierung der DVT_T0-Daten ermittelt werden.
8. In den neuen Zahnpositionen in OO_T1 beißen die Zähne im Schlussbiss anders aufeinander. Die neue Schlussbissposition wird auf die Kiefergelenke übertragen, so dass sichtbar wird, wo sich die Kiefergelenkköpfchen nun in Bezug zu den Kiefergelenkpfannen befinden. Für dieses Sichtbarmachen der neuen Situation, die sich durch die Therapie eingestellt hat, muss keine neuerliche DVT-Aufnahmen angefertigt werden.

Das nachfolgend anhand der Figuren 5 beschrieben Ausführungsbeispiel ähnelt dem ersten und bezieht sich entsprechend auf den ersten Anwendungsfall. Der Unterschied liegt darin, dass auf die Aufnahme des ersten optischen Oberflächendatensatzes OO_T0 verzichtet wird, weil hier eine feste Referenzstruktur, insbesondere nicht bewegte Zähne, gewählt werden kann, die sowohl in den Oberflächendaten als auch den DVT-Daten sichtbar ist. Unbewegte Zähne eignen sich besonders gut als feste Referenzstruktur, weil sie sich nicht verformen können und mehr geometrische Details aufweisen, die eine sichererer räumlichere Zuordnung erlauben.

In Figur 5 a ist ein DVT-Datensatz zum Zeitpunkt T0 und in Figur 5b ein optischer Oberflächendatensatz zum Zeitpunkt T1 gezeigt. Der Vergleich der Datensätze zeigt, dass nur der Zahn 4 in eine Position 4' bewegt wurde. Der Bereich 23, in dem sich die im Oberflächendatensatz ersichtlichen unbewegten Zähne 1 bis 3 befinden, kann in diesem Fall als Referenzstruktur genutzt werden.
1. Vor der Behandlung zur Zeit T0 wird lediglich ein DVT-Datensatz DVT_T0 eines Patienten z.B. im Rahmen einer Zahnstatusaufnahme aufgenommen.
2. Wenn die Behandlung zu einem Zeitpunkt T1 fortgeschritten ist, wird der okklusale Status des Patienten mit einer Intraoralkamera gescannt und ein Oberflächendatensatz OO_T1 aufgenommen, wobei diese Aufnahme wiederum ohne die KFO-Apparatur im Mund des Patienten geschieht.
3. Nachfolgend werden die Oberflächendaten OO_T1 anhand der festen Referenzstruktur 23 mit den DVT-Daten DVT_T0 registriert. Dazu werden in dem Oberflächendatensatz OO_T1 Zähne oder Kieferbereiche, die sich seit T0 nicht bewegt haben, markiert. Dies kann manuell oder durch einen Algorithmus erfolgen. Die OO_T1-Daten werden dann rigide auf die DVT_T0-Daten registriert, wobei nur Zähne oder Kieferbereiche für die Registrierung verwendet werden, die zuvor markiert wurden.
4. Nun wird die Registrierung des okklusalen Status zum Zeitpunkt T1 mit dem DVT-Datensatz DVT_T0 anhand einer teilrigiden Registrierung durchgeführt, wobei die im Oberflächendatensatz OO_T1 sichtbaren Kronen mit den DVT-Daten DVT_T0 zusammengeführt und dabei die Zahnbewegungen auf die DVT-Daten übertragen werden. Dabei werden die Bereiche in den OO_T1-Daten betrachtet, die sich seit dem Zeitpunkt T0 bewegt haben, also nicht markiert sind. Diese werden dann teilrigide, insbesondere unter Zuhilfenahme einer Segmentierung, mit den DVT_T0-Daten registriert.
5. Bei der in Schritt 4) durchgeführten teilrigiden Registrierung ist aus den OO_T1-Daten für jeden (segmentierten) Zahn der Transformationsvektor bekannt, mit dem sich dieser zwischen T0 und T1 bewegt hat.
6. Nun werden die identifizierten Transformationsvektoren der Zahn(kronen) auf die DVT_T0-Daten übertragen. Beispielsweise sei ein bestimmter Zahn zwischen dem Zeitpunkt T0 und T1 entsprechend der Transformation Transf_T0_T1 bewegt worden. Diese Transformation wird innerhalb der DVT_T0-Daten auch auf das in den Oberflächendaten unsichtbare knöcherne Gewebe unterhalb der Zahnkrone übertragen und visualisiert. Durch eine solche "Verlängerung" der Zahnkrone nach unten kann das entsprechende Wurzelgewebe des Zahnes ermittelt werden. Alternativ kann das Wurzelgewebe durch eine Segmentierung der DVT_T0-Daten ermittelt werden.
7. In den neuen Positionen in OO_T1 beißen die Zähne im Schlussbiss anders aufeinander. Diese neue Schlussbissposition wird auf die Kiefergelenke übertragen, so dass sichtbar wird, wo sich die Kiefergelenke nun befinden. Für dieses Sichtbarmachen der neuen Situation, die sich durch die Therapie eingestellt hat, muss keine neuerliche DVT-Aufnahmen angefertigt werden.

Entsprechend dem nachfolgenden dritten Ausführungsbeispiel, das sich auch auf den ersten Anwendungsfall bezieht, kann eine anatomische Verlaufskontrolle einer Therapie mit festsitzender kieferorthopädischer Apparatur durchgeführt werden. Dabei entspricht die Schrittfolge der nach dem ersten Ausführungsbeispiel, wobei die Brackets, die fest an den Zähnen sitzen und damit in den OO_T1-Daten sichtbar sind, ausmaskiert werden, bevor die OO_T1-Daten mit den OO_T0-Daten registriert werden. Dasselbe kann auch entsprechend der Schritte des oben genannten modifizierten ersten Ausführungsbeispiels geschehen, wobei die Brackets, die fest an den Zähnen sitzen und damit in den OO_T1-Daten sichtbar sind, ausmaskiert werden, bevor sie mit den DVT_T0-Daten registriert werden.

Das nachfolgend beschriebene dritte Ausführungsbeispiel betrifft den zweiten Anwendungsfall und ermöglich entsprechend die Visualisierung der anatomischen Auswirkungen anhand eines konventionellen KFO-Setups. Die nachfolgende Schrittfolge ist wiederum zumindest bezüglich einiger Schritte nicht zwingend:
1. Zunächst wird vor der KFO-Behandlung zum Zeitpunkt T0 ein dreidimensionaler DVT-Datensatz DVT_T0 des Patienten angefertigt.
2. Auf der Basis eines abgeformten Gipsmodells wird ein konventionelles KFO-Setup durchgeführt. Dazu wird die Oberfläche des Gipsmodells zunächst mit einem optischen Scanner gescannt und so der Oberflächendatensatz OO_T0 erstellt.
3. Anschließend wird das Gipsmodell derart zersägt, dass die für die Therapie entscheidenden Zähne gelöst und separat in die kieferorthopädische Zielposition gebracht werden können. In die geplante Zielposition werden die Zähne insbesondere mit einem (rot) eingefärbten Wachs fixiert.
4. Das nun vorhandene modifizierte Gipsmodell, das die Zahnstellung, die mit der Therapie erreicht werden soll, repräsentiert, wird mit einem optischen Scanner gescannt, wobei der Oberflächendatensatz OO_T1 erstellt wird. Falls der Scanner Farben erkennt, kann anhand der (roten) Farbe des Wachses der Bereich identifiziert werden, der sich geändert hat und entsprechend nicht im ursprünglichen OO_T0-Datensatz zu finden ist.
5. Der ursprüngliche Oberflächendatensatz OO_T0 wird mit den DVT-Daten DVT_T0 registriert.
6. Zudem wird der Oberflächendatensatz OO_T0 mit dem Oberflächendatensatz OO_T1 registriert. Dabei werden zunächst Bereiche, insbesondere Gingiva-Bereiche, identifiziert, die sich in der Therapie nicht verändert haben. Die (rot) gefärbten Wachs-Bereiche, in denen sich die Zahnstellung im Modell geändert hat, werden nicht berücksichtigt, ebensowenig Zähne, die von der Gingiva durch (rotes) Wachs separiert sind.
7. Anschließend werden die Bereiche, die im Modell verändert wurden, teilrigide von dem Oberflächendatensatz OO_T0 auf den Oberflächendatensatz OO_T1 registriert. Dabei kann wiederum auf eine Segmentierung zurückgegriffen werden.
8. Die so ermittelten Bewegungen der Zahnkronen werden wie in den oben beschriebenen Ausführungsbeispielen in dem DVT-Datensatz auf die Zahnwurzeln und auf die Kiefergelenke übertragen, so dass ein simulierter Volumendatensatz der am Gipsmodell geplanten Zahnumstellung entsteht. Anhand dieses simulierten Volumendatensatzes kann der behandelnde Arzt die Folgen seiner geplanten Therapie auf die Anatomie des Patienten erkennen.

Auch das nun zu beschreibende Ausführungsbeispiel betrifft den zweiten Anwendungsfall der Therapieplanung. Es liegt in diesem Fall kein Oberflächendatensatz OO_T0 von dem unveränderten Gipsabdruck vor:
1. Im Vorfeld der KFO-Behandlung zum Zeitpunkt T0 liegt ein DVT-Datensatz DVT_T0 des Patienten vor.
2. Es wird ein konventionelles KFO-Setup auf Basis eines Gipsmodells durchgeführt.
3. Das Gipsmodell wird derart zersägt, dass die für die Therapie entscheidenden Zähne gelöst und separat in die kieferorthopädische Zielposition gebracht werden können. In die geplante Zielposition werden die Zähne insbesondere mit einem (rot) eingefärbten Wachs fixiert.
4. Das nun vorhandene modifizierte Gipsmodell, das die Zahnstellung, die mit der Therapie erreicht werden soll, repräsentiert, wird mit einem optischen Scanner gescannt, wobei der Oberflächendatensatz OO_T1 erstellt wird. Fall der Scanner Farben erkennt, kann anhand der (roten) Farbe des Wachses die Bereiche identifiziert werden, die sich geändert haben.
5. Der solchermaßen erstellte Oberflächendatensatz OO_T1 wird mit den DVT-Daten DVT_T0 registriert. Dazu werden die Zähne respektive Kieferbereiche identifiziert, die sich nicht verändert haben
6. Nun werden die Bereiche, die im Zuge der Planung verändert wurden, teilrigide von OO_T1 auf DVT_T0 registriert. Dabei kann auf eine Segmentierung zurückgegriffen werden.
7. Die ermittelten Bewegungen der Zahnkronen werden auf die Zahnwurzeln in den DVT-Daten und auf die Kiefergelenke übertragen. Der simulierte DVT-Volumendatensatz, der dem Gipsmodell der geplanten Zahnumstellung zuzuordnen ist, dient dem behandelnden Arzt dazu, die Folgen seiner geplanten Therapie auf die Anatomie des Patienten abschätzen zu können.

Statt der Gipsmodelle können auch dreidimensional gedruckte Modelle genutzt werden. Der Ablauf gestaltet sich wie folgt:
1. Zunächst werden Zahnbögen des Patienten mit einem optischen Scanner aufgenommen. Zusätzlich werden in den Scandaten auf Höhe der Gingiva künstliche Erhebungen vorgesehen, die zur späteren Registrierung dienen.
2. Somit entsteht der im Verhältnis zum Original etwas modifizierte Oberflächendatensatz OO_T0.
3. Der Oberflächendatensatz OO_T0 wird mit einem 3D Drucker gedruckt.
4. Anschließend wird der anhand der OO_T0 entstandene Druck zersägt und eine Planung in Form eines Set Up erstellt. Dieser Schritt gleicht dem, der auch am Gipsmodell durchgeführt wird.
5. Der nun vorhandene modifizierte Druck, der die Zahnstellung, die mit der Therapie erreicht werden soll, repräsentiert, wird mit einem optischen Scanner gescannt und der der Oberflächendatensatz OO_T1 erstellt.
6. Der erstellte Oberflächendatensatz OO_T1 wird mit den DVT-Daten DVT_T0 registriert. Durch die in Schritt 1 eingebrachten künstlichen und eindeutig geformten Erhebungen ist eine verbesserte Registrierung zwischen den Oberflächendatensätzen OO_T0 und OO_T1 möglich. Schließlich hat die natürliche Gingiva mitunter zu wenig Details, an denen sich eine oberflächenbasierte Registrierung orientieren kann. Die künstlichen Erhebungen lassen hingegen eine sicherere absolute räumliche Zuordnung zu.

Anstatt in den zuletzt beschriebenen Ausführungsbeispielen zur Erzeugung des Oberflächendatensatzes einen optischen Scan an einem dreidimensional gedruckten Modell oder an einem Gipsmodell zu nutzen, ist es auch möglich, den Oberflächendatensatz der veränderten Zahnstellung rechnerisch aus einem vorhandenen Oberflächendatensatz der ursprünglich noch nicht veränderten Zahnstellung am Rechner durch Simulation zu erzeugen ("Digitales Setup"). In diesem Fall wird zur Visualisierung der anatomischen Auswirkungen unmittelbar ein digitales Setup eingesetzt.

Dasselbe geht nach dem oben beschriebenen Ausführungsbeispiel selbst dann, wenn vor der Behandlung zum Zeitpunkt T0 keine Oberflächendaten vorhanden sind.

Statt eines "positiven" Gipsmodells von der (geplanten) Zahnstellung, kann auch der "negative" Abdruck selber oder in Form eines kieferorthopädischen Aligners genutzt werden. Entsprechend kann die Visualisierung der anatomischen Auswirkungen anhand eines solchen Aligners oder eines Aligner-Sets in der Art einer Verlaufskontrolle geschehen. Wurde der Aligner vom Patienten noch nicht getragen, weil er beispielsweise aus einer Serie von Alignern stammt und erst in ein paar Monaten getragen werden soll, bietet das Verfahren einen Blick in die Zukunft, wie die Zahnwurzeln nach dem Einsatz des Aligners orientiert sein werden. Der Ablauf des Verfahrens gestaltet sich beispielsweise wie folgt:
1. Zunächst wird ein Oberflächendatensatz an der "negativen" Oberfläche, die beim Tragen des Aligners an den Zähnen anliegt, mit einer Intraoralkamera oder einem Laborscanner eingescannt. Diese an den Zähnen anliegende "negative" Oberfläche kann alternativ auch aus digitalen Geometriedaten eines virtuell geplanten Aligners erzeugt werden.
2. Der Oberflächendatensatz der Zahninnenflächen werden mit dem Registrierverfahren auf die Zahnkronen im DVT-Datensatz registriert.
3. Damit können die anatomischen Auswirkungen des Aligners visualisiert werden, ohne dass ein neues DVT angefertigt werden muss. Dasselbe kann auch an einer Serie von Alignern geschehen. Damit können die Auswirkungen der Aligner-Serie auf Zahnwurzeln und Kiefergelenke visualisiert werden. Das dient zur forensischen Absicherung einer KFO-Behandlung auch im Bezug auf die Kiefergelenke. Dabei ist auch eine Animation in den 3D-DVT-Daten sowie in aus den DVT-Daten berechneten Ansichten wie eine Panoramaansicht oder berechneten cephalometrischen Ansichten möglich, welche die anatomische Sequenz von der Ausgangssituation zur Zielsituation zeigt.

In einem weiteren Ausführungsbeispiel wird ein Referenzkörper eingesetzt, mit dem während des Behandlungsverlaufes die Zuordnung zwischen den CAD/CAM-Daten und den DVT-Daten ermittelt werden kann. Der Einsatz eines solchen Referenzkörpers ist hilfreich, wenn zum Zeitpunkt keine OO_T0-Daten vorhanden sind. Der Referenzkörper kommt in den Mund und wird zum Zeitpunkt OO_T1 mitgescannt. Dabei wird sichergestellt, dass der Referenzkörper immer in einem festen respektive bekannten Verhältnis zur Kieferanatomie steht, die sich während der Zahnbewegungen nicht bewegt.

Wenn der Referenzkörper an einem Zahn befestigt wird, der sich in der Zwischenzeit bewegt hat, muss der Referenzkörper beim zweiten Scan so verändert werden, dass der Hotspot des Referenzkörpers an der gleichen absoluten Position im Bezug zum Kiefer ist, wie beim ersten Scan. Das kann sichergestellt werden, wenn bekannt ist, wie sich dieser Zahn absolut bewegt hat.

Diese Vorgehensweise ist quasi das "Gegenstück" zu dem oben dargestellten Anwendungsbeispiel der Visualisierung der anatomischen Auswirkungen konventioneller KFO-Setups auf Basis von gedruckten Modellen, wobei in diesem Fall nicht an dem gedruckten Set Up sondern am Patienten eine Referenzierungshilfe angebracht wird. Die Idee liegt darin, dass der Referenzkörper zum Zeitpunkt T_0 an einem Zahn angebracht und zumindest in den Oberflächendaten mitgescannt wird. Vorteilhafterweise hat der Referenzkörper Teile, die sowohl in den Oberflächendaten als auch in den DVT-Daten gut erkennbar sind.

## Patentansprüche

1. Computergestütztes Verfahren zur Darstellung einer Veränderung einer Zahnstellung in einem Kiefer, wobei ein Volumendatensatz einer Ausgangsstellung mehrerer Zähne im Ober- oder Unterkiefer angefertigt wird,
**dadurch gekennzeichnet,**
**dass** nach einer realen oder virtuellen Veränderung der Zahnstellung ein Oberflächendatensatz angefertigt wird, der Oberflächendaten der Zähne und/oder zumindest von Teilen des vom Volumendatensatz umfassten Kiefers in der veränderten Zahnstellung enthält,
**dass** der Oberflächendatensatz mit dem Volumendatensatz vermittels einer im Oberflächendatensatz ersichtlichen Referenzstruktur, die von der Veränderung nicht betroffen ist, referenziert wird,
**dass** nach der Referenzierung die Veränderung der Zahnstellung in den Volumendatensatz rechnerisch transformiert wird, wobei aus dem Volumendatensatz ermittelte Grenzflächen der Zähne mit Oberflächendaten der entsprechenden Zähne aus dem Oberflächendatensatz in Deckung gebracht werden,
**dass** bei der Transformation im Volumendatensatz enthaltene und unter der Oberfläche verborgene Zahnstrukturen entsprechend rechnerisch bewegt werden, und
**dass** ein modifizierter Volumensatz zur Darstellung ausgegeben wird, der die rechnerisch ermittelte Veränderung der Zahnstellung umfasst.

2. Verfahren nach Anspruch 1,
**dadurch gekennzeichnet,**
**dass** der Oberflächendatensatz mit einem optischen Scanverfahren entweder nach einer Veränderung der Zahnstellung an den Zähnen oder nach einer Veränderung der Zahnstellung an einem Modell angefertigt wird.

3. Verfahren nach Anspruch 1,
**dadurch gekennzeichnet,**
**dass** der Oberflächendatensatz durch eine virtuelle Veränderung der Zahnstellung in einem ursprünglichen Oberflächendatensatz angefertigt wird.

4. Verfahren nach einem der Ansprüche 1 bis 3,
**dadurch gekennzeichnet,**
**dass** die Referenzstruktur aus dem Volumendatensatz anhand von Gewebsgrenzen umfassend Zähne und/oder Kieferabschnitte rechnerisch extrahiert wird, wobei anhand der Referenzstruktur eine Referenzierung des Volumendatensatzes mit dem Oberflächendatensatz erfolgt.

5. Verfahren nach einem der Ansprüche 1 bis 3,
**dadurch gekennzeichnet,**
**dass** zu einem ersten Zeitpunkt ein ursprünglicher Oberflächendatensatz aufgenommen und eine Registrierung zwischen dem ursprünglichen Oberflächendatensatz und dem Volumendatensatz vorgenommen wird, wobei anhand des ursprünglichen Oberflächendatensatzes und des Oberflächendatensatzes die unveränderte Referenzstruktur ermittelt wird, vermittels derer eine Registrierung zwischen dem ursprünglichen Oberflächendatensatz und dem Oberflächendatensatz durchgeführt wird, wobei die Transformation in dem Volumendatensatz anhand der ermittelten Bewegungen der Zahnkronen erfolgt.

6. Verfahren nach Anspruch 1,
**dadurch gekennzeichnet,**
**dass** der Oberflächendatensatz mit einem optischen Scanverfahren an einem Modell der Zähne erstellt wird,
wobei das Modell mittels eines Abdrucks der veränderten Zahnstellung erzeugt wird oder
wobei das Modell mittels eines Abdrucks der ursprünglichen Zahnstellung erzeugt wird, wobei aus dem so erzeugten Modell Zähne herausgenommen und in veränderter Position wieder fixiert werden.

7. Verfahren nach Anspruch 1,
**dadurch gekennzeichnet,**
**dass** der Oberflächendatensatz mit einem optischen Scanverfahren an einem Modell der Zähne erstellt wird, das mittels eines 3D Druckers erstellt wird.

8. Verfahren nach Anspruch 1,
**dadurch gekennzeichnet,**
**dass** der Oberflächendatensatz durch Scannen der die Zähne beaufschlagenden Flächen einer individuell angefertigten korrigierenden Schiene, insbesondere einem Aligner, erstellt wird.

9. Verfahren nach einem der vorherigen Ansprüche,
**dadurch gekennzeichnet,**
**dass** der Oberflächenscan am Patienten mit einem an den Zähnen und/oder am Kiefer angebrachten Referenzkörper erstellt wird.

10. System, umfassend einen Rechner und einen optischen Scanner, zur Ausführung des Verfahrens nach einem der vorherigen

## Claims

1. A computer-aided method for representing a change in a tooth position in a jaw, wherein a volume data set of an initial position of a plurality of teeth in the upper jaw or lower jaw is prepared,
**characterized in that**
a surface data set which contains surface data of the teeth and/or of at least portions of the jaw comprised in the volume data set in the changed tooth position is prepared after an actual or virtual change in the tooth position,
**in that** the surface data set is referenced with the volume data set by means of a reference structure which is evident in the surface data set and which is not affected by the change,
**in that** after the referencing, the change in the tooth position is computationally transformed into the volume data set, wherein interfaces of the teeth determined from the volume data set are brought into alignment with surface data for the corresponding teeth from the surface data set,
**in that** during the transformation, tooth structures contained in the volume data set and concealed under the surface are correspondingly computationally moved, and
**in that** a modified volume set which comprises the computationally determined change in the tooth position is output for the purpose of representation.

2. The method as claimed in claim 1,
**characterized in that**
the surface data set is prepared with an optical scanning method either after a change in the tooth position on the teeth or after a change in the tooth position on a model.

3. The method as claimed in claim 1,
**characterized in that**
the surface data set is prepared by a virtual change in the tooth position in an original surface data set.

4. The method as claimed in one of claims 1 to 3,
**characterized in that**
the reference structure is computationally extracted from the volume data set with the aid of tissue boundaries comprising teeth and/or jaw sections, wherein a referencing of the volume data set with the surface data set is carried out with the aid of the reference structure.

5. The method as claimed in one of claims 1 to 3,
**characterized in that**
at a first point in time, an original surface data set is recorded and a registration is carried out between the original surface data set and the volume data set wherein the unchanged reference structure is determined with the aid of the original surface data set and the surface data set, by means of which a registration is carried out between the original surface data set and the surface data set, wherein the transformation is carried out in the volume data set with the aid of the determined movements of the crowns of the teeth.

6. The method as claimed in claim 1,
**characterized in that**
the surface data set is generated with an optical scanning method on a model of the teeth,
wherein the model is produced by means of an impression of the changed tooth position, or
wherein the model is produced by means of an impression of the original tooth position, wherein teeth are taken out of the model produced in this manner and are replaced in the changed position.

7. The method as claimed in claim 1,
**characterized in that**
the surface data set is generated with an optical scanning method on a model of the teeth which is generated with a 3D printer.

8. The method as claimed in claim 1,
**characterized in that**
the surface data set is generated by scanning the surfaces of an individually prepared correcting brace which have an impact on the teeth, in particular an aligner.

9. The method as claimed in one of the preceding claims,
**characterized in that**
the surface scan is generated on patients with a reference structure which is attached to the teeth and/or the jaw.

10. A system comprising a computer and an optical scanner for implementing the method as claimed in one of the preceding claims.

## Revendications

1. Procédé assisté par ordinateur pour représenter une modification d'une position des dents dans une mâchoire, dans lequel un jeu de données de volume d'une position de départ de plusieurs dents dans la mâchoire supérieure et la mâchoire inférieure est produit,
**caractérisé en ce**
**qu'**un jeu de données de surfaces est produit après une modification réelle ou virtuelle de la position des dents, qui contient des données de surface des dents et/ou au moins de parties de la mâchoire comprise dans le jeu de données de volume dans la position des dents modifiée,
**que** le jeu de données de surfaces est référencé avec le jeu de données de volume au moyen d'une structure de référence visible dans le jeu de données de surface qui n'est pas touchée par la modification,
**qu'**après le référencement, la modification de la position de dents dans le jeu de données de volume est transformée par calcul, dans lequel des surfaces limites des dents déterminées à partir du jeu de données de volume sont amenées en recouvrement avec des données de surface des dents correspondantes du jeu de données de surface,
**que** lors de la transformation, des structures de dents contenues dans le jeu de données de volume et cachées sous la surface sont déplacées de manière correspondante par calcul, et
**qu'**un jeu de volume modifié est émis pour être représenté, lequel comprend la modification de la position des dents déterminée par calcul.

2. Procédé selon la revendication 1,
**caractérisé en ce que**
le jeu de données de surface est produit par un procédé de numérisation optique soit après une modification de la position des dents sur les dents, soit après une modification de la position des dents sur un modèle.

3. Procédé selon la revendication 1,
**caractérisé en ce que**
le jeu de données de surface est produit par une modification virtuelle de la position des dents dans un jeu de données de surface d'origine.

4. Procédé selon l'une des revendications 1 à 3, **caractérisé en ce que**
la structure de référence est extraite par calcul du jeu de données de volume à l'aide de limites tissulaires comprenant des dents et/ou des sections de mâchoire, dans lequel, un référencement du jeu de données de volume avec le jeu de données de surface est effectué à l'aide de la structure de référence.

5. Procédé selon l'une des revendications 1 à 3,
**caractérisé en ce**
**qu'**à un premier moment, un jeu de données de surface d'origine est enregistré et qu'un enregistrement entre le jeu de données de surface d'origine et le jeu de données de volume est effectué, dans lequel la structure de référence non modifiée est déterminée à l'aide du jeu de données de surface d'origine et du jeu de données de surface, au moyen de laquelle un enregistrement entre le jeu de données de surface d'origine et le jeu de données de volume est effectué, dans lequel la transformation s'effectue dans le jeu de données de volume à l'aide des mouvements des couronnes dentaires déterminés.

6. Procédé selon la revendication 1,
**caractérisé en ce que**
le jeu de données de surface est réalisé avec un procédé de numérisation optique sur un modèle des dents,
dans lequel le modèle est produit au moyen d'une empreinte de la position de dents modifiée ou
dans lequel le modèle est produit au moyen d'une empreinte de la position de dents d'origine, dans lequel des dents sont extraites et repositionnées dans une position modifiée à partir du modèle ainsi produit.

7. Procédé selon la revendication 1,
**caractérisé en ce que**
le jeu de données de surface est produit par un procédé de numérisation optique sur un modèle des dents qui est produit au moyen d'une imprimante 3D.

8. Procédé selon la revendication 1,
**caractérisé en ce que**
le jeu de données de surface est réalisé par numérisation des surfaces sollicitant les dents d'un rail de correction produit individuellement, en particulier un aligneur.

9. Procédé selon l'une des revendications précédentes,
**caractérisé en ce que**
la numérisation des surfaces est réalisée sur le patient avec un corps de référence appliqué sur les dents et/ou la mâchoire.

10. Système, comprenant un ordinateur et un scanner optique pour exécuter le procédé selon l'une des revendications précédentes.
